# EUROPEAN PATENT APPLICATION

(11) **EP 3 734 282 A1**
(43) Date of publication of application: **04.11.2020**
(21) Application number: 18894064.7
(22) Date of filing: 14.11.2018
(51) Int. Cl.: G01N 33/68

(54) **APPARATUS, REAGENT KIT, AND METHOD FOR DETECTING MISFOLDED PROTEIN**

(30) Priority: 28.12.2017 CN 201711468345; 28.12.2017 CN 201721889634 U; 20.09.2018 CN 201811117548
(71) Applicant: Shuwen Biotech Co., Ltd., Huzhou, Zhejiang 313200 (CN)
(72) Inventor: LI, Xingmin, Huzhou, Zhejiang 313200 (CN); XU, Jun, Huzhou, Zhejiang 313200 (CN); TANG, Xiaobin, Huzhou, Zhejiang 313200 (CN); ZHANG, Jay Zhe, Huzhou, Zhejiang 313200 (CN)
(74) Representative: HGF
(86) International application number: PCT/CN2018/115444
(87) International publication number: WO 2019/128506

(57) **Abstract**

The invention discloses a device, a kit and a method for determining whether a biological sample contains misfolded protein or misfolded protein aggregates. The detection device comprises a testing component and a sample applicator, the testing component comprises one or more microporous membranes, and the sample applicator comprises one or more capillary tubes. The kit comprises a dye that is capable of binding to misfolded proteins and microporous membranes. During the detection, the sample to be tested is mixed with the dye to form a mixture. The mixture is taken up by a capillary tube. The liquid outlet of the capillary tube is then place in close contact with the surface of a microporous membrane, allowing the mixture to be drawn from the capillary tube into the microporous membrane. The presence or absence of misfolded proteins in the test sample is determined based on the diffusion of the dye in the membrane, as observed by naked eyes or using an instrument.

## Description

### Related Applications and Priority

This application is a national phase entry of PCT/CN2018/115444 filed on November 14, 2018 claiming the benefit of Chinese Application No. 201711468345.X and Chinese Application No. 201721889634.2, both filed on December 28, 2017 and Chinese Application No. 201811117548.9, filed on September 30, 2018. The entire eachings of the referenced applications are incorporated herein by reference.

### Field of the Invention

The present invention generally relates to devices, kits and methods for detection of biomaterials, specifically to devices, kits and methods for determining whether a biological sample contains misfolded proteins.

### Background of the Invention

The natural protein folding process is complex and error-prone, and often gives rise to protein misfolding. Misfolding is caused by an incorrect folding process that results in the formation of a protein with a different conformation from its native one.

Protein misfolding results in a large number of β-sheet structures arranged in opposite directions. The most frequent destiny for misfolded proteins is self-aggregation due to the exposure of hydrophobic protein surfaces that are normally buried inside the proteins, providing a high degree of stickiness to the β-sheets of other misfolded proteins. This results in a nucleus for further aggregation of an almost unlimited number of even unrelated misfolded polypeptide chains. Thus, misfolded proteins often exist as oligomers and fibrous aggregates.

These aggregates formed by misfolded proteins have been found to be associated with a variety of aging-related neurodegenerative diseases such as Alzheimer's disease, Parkinson's, etc. Even though these proteins are different (amyloid β peptide of Alzheimer's disease, α-Synuclein protein of Parkinson's disease, Huntingtin protein of Huntington's disease, and Prion protein of Mad cow disease), upon misfolding they all exhibit a β-sheet structure, and can form the above aggregates with the same conformation.

Recent studies have found that preeclampsic pregnant women have large amounts of misfolded proteins and aggregates in their urine and placental tissues, whereas normal pregnant women do not have such aggregates. Thus the onset of preeclampsia and eclampsia are related to these misfolded proteins and aggregates. (Buhimischi et al., Science Translational Medicine, 2014, 6(245): 245ra92). However, there is no suitable point-of-care detection kit based on this biomarker to rapidly diagnose preeclampsia.

### Summary of the Invention

In order to solve at least one of the above technical problems, a first aspect of the present invention provides a device for determining whether a biological sample contains misfolded proteins. The device includes a testing component and a sample applicator. The testing component comprises a microporous membrane, and the sample applicator comprises 1, 2, 3 or more capillary tubes. Further, the testing component comprises a first cover panel and a first bottom panel, the first cover panel having a microporous membrane.

Further, the device comprises a sample containing component having one or more sample wells therein for containing samples (test samples, negative control samples, and/or positive control samples) and/or dye.

In some embodiments of this invention, the surface of said first cover panel is covered with at least one microporous membrane.

In some embodiments of this invention, said first cover panel comprises at least one testing trough having therein a microporous membrane.

According to this invention, optionally, the sample applicator includes a hollow box-like member containing 1, 2, 3 or more capillary tubes; alternatively, the sample applicator includes a solid box-like member provided therein 1, 2, 3 or more recessed holes for anchoring respectively 1, 2, 3 or more capillary tubes. Optionally, the sample applicator is a sample applying member comprising a plate-like body portion and 1, 2, 3 or more capillary tubes disposed on the side. One end of the capillary tubes is the liquid outlet drawing in and releasing out liquid solutions, and the other end is connected to the body portion of the sample applying member; optionally, the sample applicator is a capillary tube.

A second aspect of this invention provides a kit for determining whether a biological sample contains misfolded proteins, comprising any one of the device of the first aspect described above, as well as a dye capable of binding a microporous membrane and a misfolded protein. Preferably, the dye is selected from the group consisting of a heterocyclic dye, Congo Red, thioflavin, and Evans Blue, and more preferably, the dye is Congo Red.

A third aspect of the invention provides a kit for determining whether a biological sample contains misfolded proteins, comprising a microporous membrane and 1, 2, 3 or more capillary tubes. In an embodiment of the present invention, the kit further comprises a dye capable of binding a microporous membrane and a misfolded protein, and preferably, the dye is selected from the group consisting of a heterocyclic dye, Congo Red, thioflavin, and Evans Blue, and more preferably, the dye is Congo Red.

Further, the above kit may include control samples, such as a negative control sample and/or a positive control sample.

A fourth aspect of this invention provides a method of determining whether a biological sample contains misfolded proteins, comprising the steps of:
mixing the sample with a dye capable of binding to a microporous membrane and misfolded proteins, to form a mixture;
drawing at least 5 µL of the mixture with a capillary tube;
placing the liquid outlet of the capillary tube in full and close contact with the surface of the microporous membrane to slowly and completely release the mixture in the capillary tube into the microporous membrane; and
determining whether the sample contained misfolded proteins by observing the diffusion of the mixture in the microporous membrane based on the color of the dye.

Preferably, the mixture is drawn into the capillary tube through capillary action. Preferably, 5 to 25 µL, more preferably 8 to 15 µL of said liquid mixture is drawn into the capillary tube and completely released into the microporous membrane.

A fifth aspect of the invention provides an assembly for determining whether a biological sample contains misfolded proteins, comprising a capillary tube and a microporous membrane, wherein the liquid outlet of the capillary tube is in full and close contact with the microporous membrane.

Further, the capillary tube may contain a certain amount of mixture formed by mixing a biological sample with a dye capable of binding a microporous membrane and misfolded proteins. And the liquid outlet of the capillary tube is in close contact with the microporous membrane. Preferably, the dye is selected from one or more of the group consisting of a heterocyclic dye, Congo Red, thioflavin, and Evans Blue, more preferably, the dye is Congo Red. A certain amount means 5 µL or more, preferably 5 to 25 µL, more preferably 8-15 µL.

Further, in one embodiment, the surface of the microporous membrane surrounding the liquid outlet is stained after a certain amount of the mixture formed by mixing a biological sample with Congo Red, is released from the capillary tube.

### Brief description of the Drawings

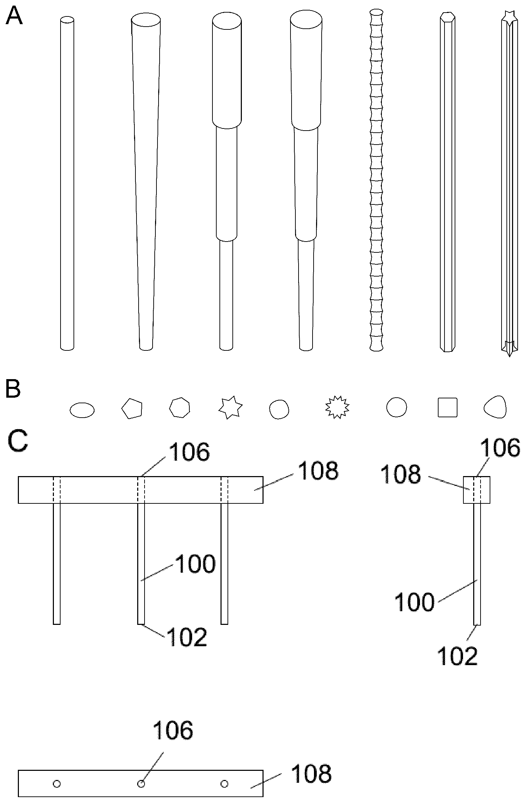

Figure 1A shows some types of capillary tubes used in this present invention (A: capillary tubes with various shapes, from left to right: A capillary tube of even thicknesses longitudinally; a capillary tube, with its thicknesses decreasing gradually from one end to the other; a capillary tube with several segments having same thickness; a capillary tube having several segments of different thickness and the thickness of each segment gradually decreasing from one end to the other end; a capillary tube with a wave-like shape; a capillary tube with a pentagonal cross section; a capillary tube with a cross section of pentagram shape.) Figure 1B shows a selection of other cross section shapes of the end of a capillary tube that contacts with the microporous membrane. Figure 1C shows a schematic view of a sample applicator with three capillary tubes. The upper left part is a front view, the upper right part is a top view, and the lower part is a left view.
Figure 2 is a diagram showing a specific structure of a testing component of the present invention.
Figure 3 is another diagram showing another specific structure of a testing component of the present invention.
Figure 4 shows the placements of the microporous membrane (A, C, and E are schematic views of the placements, respectively, and B, D, and F are the corresponding cross-sectional views, respectively).
Figure 5 is a diagram showing a specific structure of the device of the present invention (A: top view, B: front view, C: left view).
Figure 6 is a diagram showing another specific structure of the device of the present invention (A: top view, B: front view, C: left view).
Figure 7 is a diagram showing another specific structure of the device of the present invention (A: top view, B: front view, C: left view).
Figure 8 is a diagram showing another specific structure of the device of the present invention.
Figure 9 is a diagram showing another specific structure of the device of the present invention.
Figure 10 is a diagram showing another specific structure of the device of the present invention (A: top view, B: front view, C: left view).
Figure 11 is a diagram showing another specific structure of the device of the present invention (A: top view, B: front view, C: left view).
Figure 12 is a diagram showing another specific structure of the device of the present invention.
Figure 13 is a diagram showing another specific structure of the device of the present invention (A: top view, B: front view, C: left view).
Figure 14 is a diagram showing the method to use a specific structure of the device of the present invention.
Figure 15 shows exemplary detection results of a device or a kit of the present invention.
Figure 16 is a schematic view showing the detection using an applicator with multiple capillary tubes of the present invention.
Figure 17 shows the effect of different manners of sample application by capillary tube, pipette or dropper on the blot diffusion.
Figure 18 shows the effect of diameter of the capillary tube on the blot diffusion.
Figure 19 shows the effect of the amount of the mixture applied on the blot diffusion.
Figure 20 is a schematic view showing an automatic determination system of the present invention.

### Detailed Description of the Invention

### Definition of Terms

The term "misfolded protein" as used herein is relative to a correctly folded protein. The most frequent destiny of misfolded proteins is self-aggregation due to the exposure of hydrophobic protein surfaces that are normally buried inside the proteins, resulting in a high degree of stickiness. The β-sheet structural motif offers the most favorable organization for these intermolecular aggregates, and as a result misfolded proteins often exist as aggregates (oligomers and fibrous aggregates). In the present invention, the meaning of "misfolded protein" includes such aggregates formed by misfolded proteins.

For example, a study found that the urine and placental tissue contain a large number of misfolded proteins and their aggregates in preeclampsic patients, which can specifically bind to Congo red, and when observed under electron microscope, these congophilic misfolded protein aggregates have a fibrous structure very similar to amyloid-like, whereas in normal pregnant women there is no such aggregate (Buhimschi et al., SCIENCE Translational Medicine, 2014, 6(245):245ra92).

Once the conformation occurs and the misfolding cannot be repaired or eliminated, different proteins even with no homology in structure and sequence can form a β-sheet structure. The β-sheet structures of different proteins can interact to form aggregates containing different misfolded proteins, which can bind specifically to Congo Red.

The term "microporous membrane" as used herein means a membrane made of a microporous material or a surface covered with a microporous material, which has such a function due to its spatial structure and/or compositional specificity: capable of competing with misfolded proteins for binding to a dye. In other words, if the biological sample does not contain misfolded proteins, the dye binds to the microporous material, making it impossible or difficult for the dye to diffuse in the microporous membrane along with the solvent, thus forming a smaller colored blot. In contrast, if the dye molecules are preincubated with and are bound to misfolded proteins, they cannot bind to the microporous materials. Consequently, the dye that is bound to misfolded proteins can diffuse in the microporous membrane, resulting in a larger colored blot. The microporous material can be any material known to those skilled in the art that contains a large amount of free hydroxyl groups, such as cellulose. Thus the microporous membrane may be a cellulose membrane, such as a filter paper. The dye may be any dye of the above characteristics well known to those skilled in the art and capable of conformation-specific binding to the misfolded proteins, such as Congo Red.

The term "capillary tube" as used herein means a hollow tube having a liquid outlet with a diameter of no more than 3.5mm or a cross-sectional area of no more than 9 mm², and when the capillary is inserted into the liquid, the liquid can be drawn into the capillary tube, resulting the liquid surface in the capillary tube to be higher than the external liquid, that is, a "capillary action" occurs. When the capillary tube is taken off the liquid, the liquid remaining inside the capillary tube due to the surface tension is not less than 5 µL. Preferably, the inner surface of the capillary is hydrophilic.

The material and texture or treatment of the capillary tube can be those understood by skilled in the art enabling the capillary tube to reach the volume of the liquid described above.

In the process of detecting misfolded proteins based on the capillary-based blot diffusion method in microporous membrane, the diffusion area of a dye in a microporous membrane such as filter paper is used to determine the presence or absence of misfolded proteins. The dye is capable of conformation-specifically binding to misfolded proteins, such as Congo Red dye,

However, the inventors have discovered that if a pipette (such as a dropper, a pipetteman, etc.) is used for pipetting, the liquid droplets are directly dropped onto the filter paper, forming a large contact surface with the filter paper and then rapidly diffusing in the filter paper. Thus even negative samples without misfolded proteins can form large colored stains on the filter paper that are not significantly different from the colored blots formed by positive samples, resulting in inaccurate determinations, including false positives and false negatives. This defect cannot be overcome even if the solution outlet (or tip) of the dropper or pipette tip used is small. The inventors further discovered that the size difference between the colored blots formed by negative samples and positive samples are significantly increased, the false positive rate is greatly reduced, and the detection accuracy is significantly improved, if samples are applied in a capillary tube by contacting (rather than by dropping from the air). That is, after a sufficient volume of the liquid mixture of a dye and a biological sample is drawn into a capillary tube, the orifice of the capillary tube is in full and close contact with the surface of a filter paper such that the liquid mixture in the capillary tube is slowly released into the filter paper due to absorption by the filter paper..

### Detection Method and Assembly

The present invention provides a detection method of determining whether a biological sample contains misfolded proteins or misfolded protein aggregates, comprising the steps of:
mixing the biological sample with a dye to form a mixture, and the dye is capable of binding to a microporous membrane and misfolded proteins;
drawing a certain amount of the mixture with a capillary tube;
placing the orifice of the capillary tube in full and close contact with the surface of the microporous membrane to slowly release the mixture in the capillary tube into the microporous membrane;
determining whether the biological sample contained misfolded proteins or misfolded protein aggregates by observing the diffusion of the mixture on the microporous membrane based on the color of the dye. Preferably, when the diffusion area exceeds a reference value, it is determined that the sample contains misfolded proteins or misfolded protein aggregates.

In another aspect, the present invention provides an assembly or a device for determining whether a biological sample contains misfolded protein aggregates, which can be used to carry out the above detection methods. Generally, the assembly comprises a capillary tube and a microporous membrane, wherein the liquid outlet of the capillary tube is in full and close contacted with the surface of the microporous membrane.

Specifically, the methods and the assemblies of the present invention may be used to detect a variety of biological samples, such as whole blood, serum, plasma, urine, saliva, sweat, cerebrospinal fluid, hydrothorax, tears, vaginal secretions, semen, tissue lysates and combinations thereof. When a sample contains blood, it can be centrifuged to remove red blood cells or other interfering factors before use. In specific embodiments, the methods and assemblies of the present invention are especially useful for detecting whether a pregnant woman's urine sample contains misfolded proteins or misfolded protein aggregates, thereby predicting, detecting, screening or diagnosing whether the pregnant woman has preeclampsia or has a risk of preeclampsia.

Preferably, the microporous membrane in the present invention may be a membrane made of a hydrophilic material containing free hydroxyl groups. In preferred embodiments, the microporous membrane is a cellulose membrane made of cellulose containing free hydroxyl groups, such as a filter paper, a writing paper, a printing paper, or a label paper.

The dye may be any dye capable of conformation-specific binding to misfolded proteins, while such dye should also competitively bind to the material in the microporous membrane (e.g., cellulose containing free hydroxyl groups). In preferred embodiments, the dye is Congo Red. In other embodiments, the dye is thioflavin or Evans blue. The dye may be a solid dye to be directly mixed with a liquid sample such as urine and then dissolved in such liquid sample, or may be a dye solution to be mixed with a biological sample such as urine. The concentration of the dye such as Congo Red may be 0.01 to 2 mg/mL in the resulting mixture, preferably 0.02 to 1 mg/mL, more preferably 0.05 to 0.5 mg/mL.

In the present invention, the capillary tube has two ends along the longitudinal direction of the tube, one end is an orifice for drawing or releasing liquid, the other end may be open or closed. The inner diameter of the orifice of the capillary tube is no greater than 3.5 mm or the cross-sectional area of the orifice is no more than 9 mm². The capillary tube should have a sufficient length to be capable of drawing and holding at least 4 µL of a dye-sample liquid mixture, preferably at least 5 µL or at least 8 µL, more preferably 8 to 15 µL. In some specific embodiments of the invention, the volume of the mixture in a capillary tube is 2 to 30 µL, 4 to 30 µL, more preferably 5 to 25 µL, 5 to 20 µL, 4 to 17 µL, or 5 to 17 µL, for example 5, 6, 7, 8, 9, 10, 11, 12, 13, 14, 15 or 16 µL; more preferably 8 to 16 µL. Preferably, the capillary tube is substantially uniform in thickness along the length of the tube, and the cross-section of the orifice may be circular or any other regular or irregular shape, such as those shown in Figure 1A. In preferred embodiments, the orifice has an inner diameter of about 0.5 to 3 mm, 0.7 to 3 mm, preferably 0.9 to 2.8 mm, or a cross-sectional area of about 0.2 to 7 mm², preferably about 0.64 to 6.2 mm². In a preferred embodiment, the capillary tube can draw at least 5 µL of the mixture by capillary action. In a preferred embodiment, after the capillary tube draws the mixture, the orifice is in full and close contact with a microporous membrane such that the liquid mixture is released from the capillary tube into the microporous membrane by absorption. Preferably, the rate of the release of the liquid mixture is no more than 4.5 µL/second, or no more than 4 µL/second, preferably 0.5 to 4 µL/second, for example 0.5, 1, 1.5, 2, 2.5, 3, 3.5, or 4 µL/second, more preferably 1 to 3 µL/second, such as 1.2, 1.6, 1.7, 1.8, 2.3, 2.6 or 2.8 µL/second.

If a biological sample does not contain misfolded proteins or misfolded protein aggregates, the detection result obtained by the methods, assemblies, devices or kits of the present invention is negative. That is, when the mixture of a negative sample and the dye is released into the microporous membrane by a capillary tube, no significant diffusion blot is formed on the microporous membrane, or in some embodiments a smaller diffusion blot is formed and the radius of the diffusion blot is less than a reference value, which may be determined by the maximum value of the radiuses of the diffusion blots formed by a certain number (e.g., 50-100) patient samples that clinically determined to be negative.

If a biological sample contains misfolded proteins or misfolded protein aggregates, the detection results obtained by the methods, assemblies, devices or kits of the present invention are positive. That is, when the mixture of a positive sample and the dye is released into the microporous membrane by a capillary tube, a larger diffusion blot is formed on the microporous membrane. In some embodiments of the invention, the positive sample produces a larger diffusion blot having a radius greater than or equal to a specific reference value, which may be determined by the minimum value of the radiuses of the diffusion blots formed by a certain number (e.g., 50-100) patient samples that clinically determined to be positive. It is worth noting that in some cases, a pseudopodium- or pseudopodia-like diffusion blot is produced with a radius larger than the radius of the diffusion blot formed by a negative sample, or greater than the above reference value. Even so, this type of diffusion result is still classified to be negative.

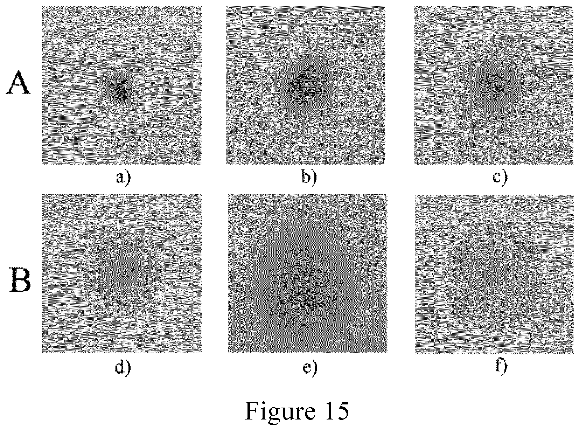

In a preferred embodiment, determining whether a biological sample contains misfolded protein or misfolded protein aggregates comprises comparing the diffusion result of the mixture in a microporous membrane to a reference card printed with examples of diffusion results of at least one negative sample and at least one positive sample. Preferably, the reference card is printed with at least 1, 2 or 3 examples selected from those shown in Figure 15A, and at least 1, 2 or 3 examples selected form those shown in Figure 15B; more preferably, the reference card is printed with all the 6 examples shown in Figure 15.

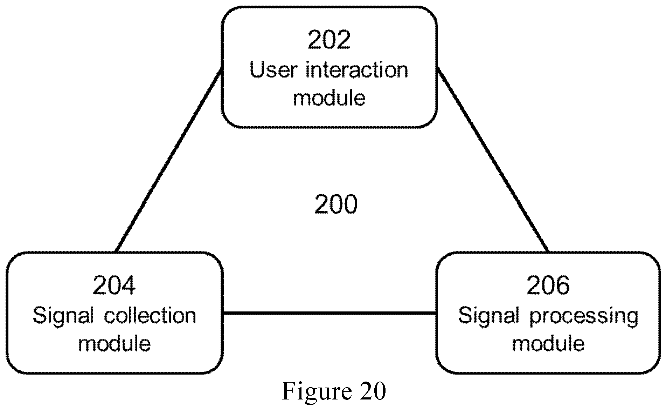

In a preferred embodiment, determining whether a biological sample contains misfolded proteins or misfolded protein aggregates comprises determining the result of the diffusion of the liquid mixture of the sample and a suitable dye in a microporous membrane by an automatic determination system to complete the detection and output the determination. For example, as shown in Figure 20, the automatic determination system 200 includes a signal collection module 204 and a signal processing module 206. Preferably, the automatic determination system 200 may further include a user interaction module 202, wherein the modules 202, 204 and 206 are connected to each other by wire or wirelessly to transmit data or signals. The signal collection module 204 comprises an optical signal collector, such as a digital camera or scanner. In use, the user sends a signal collection command to the signal collection module 204 through the user interaction module 202, and the signal collection module 204 obtains a image signal of the diffusion by photographing or scanning the diffusion result, and then transmits the collected image signal to the signal processing module 206, which compares the collected image signal with a reference database, and obtains the determination result based on a specific algorithm and transmits the result to the user interaction module 202. Preferably, the reference database contains a large amount of clinical sample determination data, and the signal processing module performs comparison by an intelligent algorithm. Optionally, the signal processing module 206 transmits the determination results to a third party system, such as a hospital's HIS or LIMS system.

Accordingly, the present invention provides an automatic determination system for determining whether a sample (e.g., a pregnant woman's urine) contains misfolded proteins or misfolded protein aggregates or whether a pregnant woman has preeclampsia or has a risk of preeclampsia. The system includes (1) an assembly, device or kit of the present invention and the above automatic determination system. In another embodiment, the automated determination system of the present invention comprises (1) an assembly, device or kit of the invention, and (2) a signal collection module having an optical signal collector, such as a digital camera or scanner, and 3) a signal memory module for storing processed or unprocessed signals collected by the signal collection module, and the signal memory module may be a HIS system or a LIMS system. An optical signal collector of the signal collection module, such as a digital camera or scanner, transmits the collected signal, i.e., the diffusion result of the mixture in the microporous membrane (e.g., in the form of image or digital information) to an HIS system or LIMS system or computer system.

In an embodiment of the assembly of the present invention described above, the orifice and a portion thereabove of the capillary tube are filled with the above described liquid mixture formed by mixing a biological sample, e.g. urine, and a dye capable of competing with a microporous membrane for binding to misfolded proteins. Preferably, the volume of the mixture is 1 to 30 µL, more preferably 1 to 25 µL, for example, about 1 to 5, 6, 7, 8, 9, 10, 11, 12, 13, 14, 15 or 16 µL, more preferably 8 to 15 µL.

Further, in the above embodiment, as the capillary tube is in full and close contact with the microporous membrane, a certain amount of the mixture contained in the capillary tube is slowly released into the microporous membrane, thereby resulting in a diffusion blot described above on the surface of the microporous membrane around the orifice of the capillary tube. Examples of diffusion blots include those shown in Figure 15. In one embodiment, after the mixture is released from the capillary tube, the dye accumulates around the orifice without diffusion and forms a dark-colored blot. In another embodiment, after the mixture is released from the capillary tube, the dye forms a dark-colored blot with "pseudopodia" around the orifice. In another embodiment, after the mixture is released from the capillary tube, the dye forms a light-colored blot with " pseudopodia" around the orifice. In another embodiment, after the mixture is released from the capillary tube, the dye forms a small red blot with distinct radius diffusion around the orifice, or forms an irregular diffusion blot without "pseudopodium" around the orifice, or forms a uniform, larger circular diffusion blot.

In a preferred embodiment of the detection method of the present invention, the method for determining whether a biological sample contains misfolded proteins or misfolded protein aggregates comprises the following steps:
mixing the biological sample with a dye capable of competing with the microporous membrane to bind misfolded proteins to form a first mixture;
drawing a certain amount of the first mixture with a first capillary tube, and placing the orifice of the first capillary tube in full and close contact with a first microporous membrane to release the first mixture in the first capillary tube into the first microporous membrane;
drawing a certain amount of a negative control sample, i.e. a second mixture, with a second capillary tube, and placing the orifice of the second capillary tube in full and close contact with a second microporous membrane to release the second mixture in the second capillary tube into the second microporous membrane;
drawing a certain amount of a positive control sample, i.e. a third mixture, with a third capillary tube, and placing the orifice of the third capillary tube in full and close contact with a third microporous membrane to release the third mixture in the third capillary tube into the third microporous membrane.

Further, whether the sample contains misfolded proteins or misfolded protein aggregates is determined by observing the diffusion of the first, second and third mixture on the microporous membranes based on the color of the dye.

The second mixture may be a dye solution determined to be free of misfolded proteins, or a mixture of a dye and a biological sample determined to be free of misfolded proteins. The third mixture may be a dye solution determined to contain misfolded proteins, may be a mixture of a dye and a positive sample determined to contain misfolded proteins, or a mixture of a dye and a misfolded protein reference sample. In preferred embodiments, the above method can simultaneously determine a plurality of (2, 3, 4, 5, 10 or more) samples, that is, the plurality of samples are separately mixed with the dye to form mixtures, and then respectively form diffusion blots by applying onto the microporous membranes by capillary tubes. The samples are determined to contain, or not to contain, misfold proteins based on the diffusion blots.

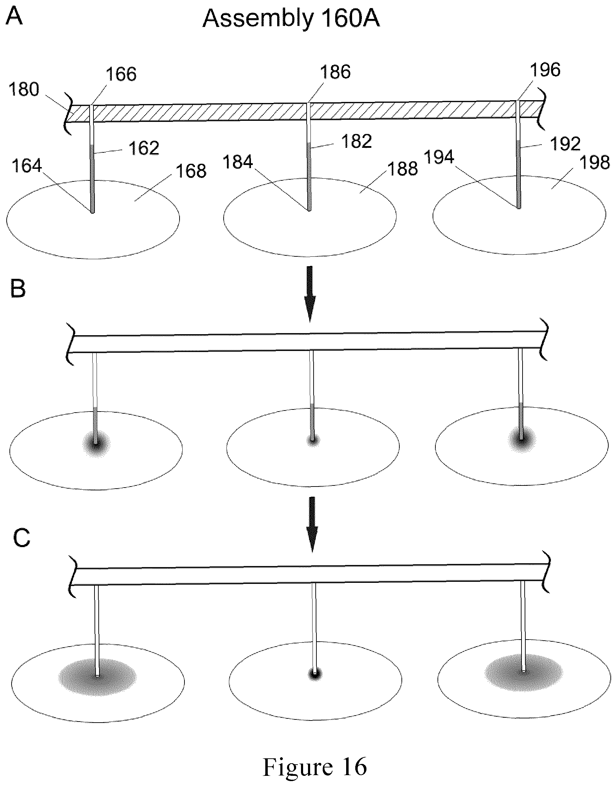

Similarly, the assembly of the present invention may comprises 2, 3, 4, 5, 6, 7, 8, 9, 10, 12 or more capillary tubes having their orifices in full and close contact with one or more microporous membranes. Those capillary tubes can be fixed together by a connecting beam to form an applicator composed of a row of capillary tubes. As shown in Figure 16, in Figure 16A, the assembly 160A comprises capillary tubes 162, 182 and 192 having orifices 164, 184 and 194, respectively, and having another opening, 166, 186, and 196, respectively. The opening 166, 186, and 196 penetrate the connecting bridge or beam so that both ends of the capillary tube are open. The connecting bridge 180 serves as a support and can fixes capillary tubes 162, 182 and 192. The orifices 164, 184, and 194 of the assembly 160A are in full and close contact with the surfaces of the microporous membranes 168, 188, 198, respectively. As shown in Figure 16A, each of the capillary tubes 162, 182, and 192 contains a liquid mixture. Preferably, the mixture in the capillary tube 162 is formed by mixing a dye with a urine sample of a pregnant woman to be tested. The liquid mixture in the capillary tube 182 is a negative control sample or is formed by mixing a dye with a buffer. While the liquid mixture in capillary tube 192 is formed by mixing a dye and a positive control sample.

Figure 16B shows that a portion of the mixture in the capillary tubes of the assembly of Figure 16A above is absorbed by the microporous membranes. Figure 16C shows that almost all the mixture in the capillary tubes of the assembly of Figure 16A above is absorbed by the microporous membranes. Thus different diffusion blots are formed on the microporous membrane.

### The Detection Device

Another aspect of the invention provides a device for determining whether a biological sample contains misfolded proteins or misfolded protein aggregates.

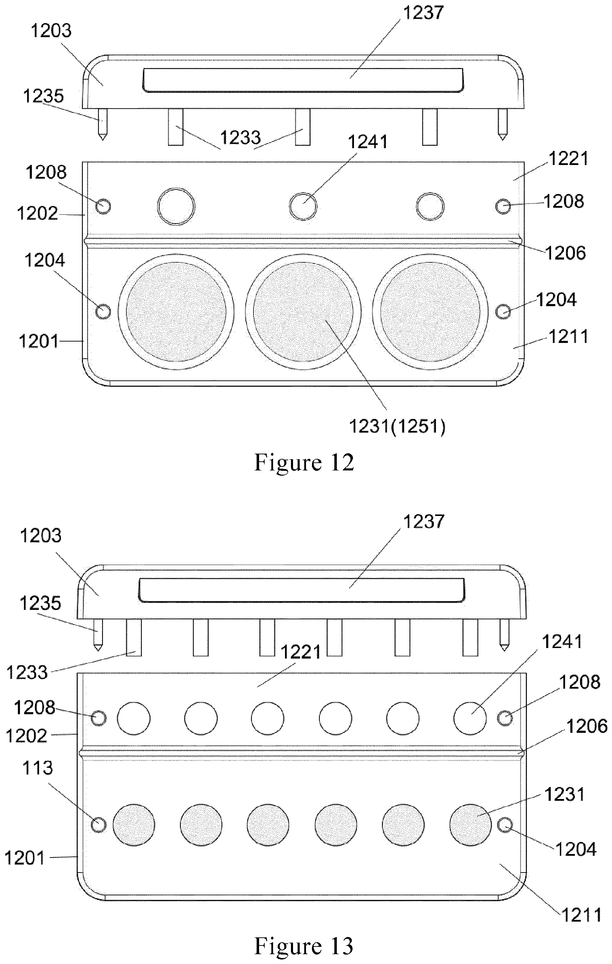

Incorporating and referring to the above description and Figures 12, 13, and 14, a specific embodiment of the present invention may provide a plate member having therein at least a first row of wells and a second row of troughs, and an applicator embedded in and operably detachable from the plate member. The number of the wells or the number of the troughs is 1 or more (3, 10, 12 or greater). The wells are sample wells and the troughs are testing troughs. The diameter of each sample well is 0.3 to 1 cm, most preferably 0.5 cm. The depth of each sample well is 0.3 to 1 cm, most preferably 0.5 cm. Each sample well can hold 30-500 µL or more of liquid. Preferably the plate member comprises 3 or more sample wells, include at least one test sample well, one negative control sample well and one positive control sample well. The test sample well can be filled with a dye above or a dye solution above or a mixture of a dye and a test sample. The negative control sample well can be filled with a negative control sample. The positive control sample well can be filled with a positive control sample. The sample wells can be sealed with a sealing sheet. The diameter of each testing trough is 0.5 to 2 cm, most preferably 1.8 cm. The depth of each testing trough is 0.2 to 1 cm, most preferably 0.3 cm. The bottom of each testing trough is covered with a microporous membrane such as filter paper. In general, the number of the testing troughs is the same or more than that of the sample wells. The applicator embedded in and operably detachable from the plate member is described as follows. Specifically, the applicator comprises one or more capillary tubes fixed on a beam or a column. For example, two or more capillary tubes are fixed to a beam in parallel to each other, and the spacing between any two adjacent capillary tubes on the beams is equal to the spacing between any two adjacent sample wells and the spacing between any two adjacent testing troughs, thereby the plurality of capillary tubes can be simultaneously inserted into the correspondingly sample wells or the correspondingly testing troughs.

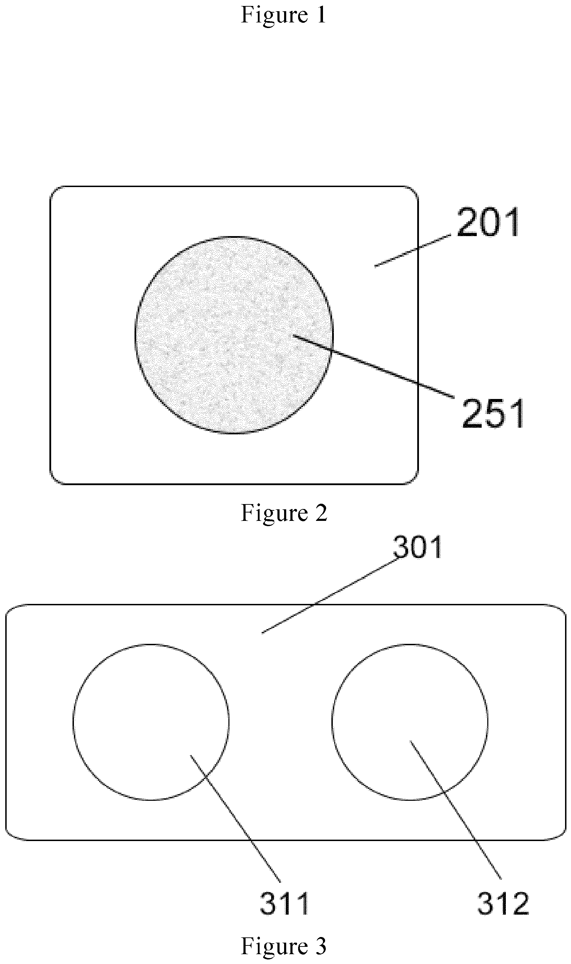

For example, the detection device of the present invention shown in Figure. 2 includes a testing component and a sample applicator. The testing component includes one or more microporous membranes, and the sample applicator has one or more capillary tubes. Preferably, the number of capillary tubes is 2 or greater, for example, 2, 3, 4, 5, 6, 7, 8, 9, 10 or greater.

In some embodiments of the invention, the testing component can be a separate microporous membrane alone.

In still other embodiments of the invention, the testing component includes a first cover panel and a first bottom panel. The first cover panel is detachably or non-detachably connected with the first bottom panel. In some embodiments, the first cover panel is non-detachably connected with the first bottom panel, and the first cover panel and the first bottom panel form an integrated panel but still artificially distinguished for the convenience of description.

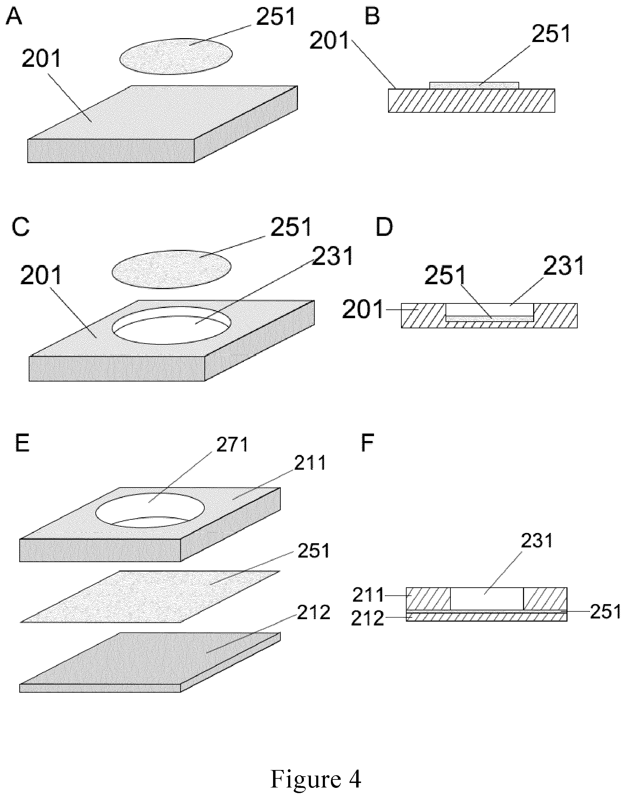

In some embodiments, the surface of the first cover panel is covered with one microporous membrane, as shown in Figures 4A or 4B. Optionally, the surface of the first cover panel is covered with one or more, for example 2, 3, 4, 5, 10, 20, 50, 100 or 200 microporous membranes. The microporous membrane may be of any shape, for example, a round, oval, square, or irregular shape. Optionally, one microporous membrane can be used for only one sample to diffuse in. More preferably, the microporous membrane is circular and has an area not less than the largest area that a sample can diffuse into. Optionally, one microporous membrane can be used for a plurality of, such as 2, 3, 4, 5, 10, 20, 50, 100, 200 or more, samples to diffuse in. More preferably, the microporous membrane can be divided into a plurality of sections each of which can be used for one sample to diffuse in. More preferably, each section is square in shape with an area of the inscribed circle of no less than the largest area that a positive sample can diffuse in.

In a specific embodiment of the present invention, the surface of said first cover panel is provided with cylindrical testing troughs with a depth of 1/3 to 2/3 of the thickness of the testing component. The bottom of each testing trough is flatly covered by a microporous membrane as shown in Figures 4C and 4D, wherein the microporous membrane is round-shaped with a diameter of slightly smaller than that of the cross section of the testing trough. In a specific embodiment, the number of testing troughs is plural, such as 2, 3, 5, 10, 15, 20, 30, 50, 100, 200, or more. Preferably, one testing trough is used for one sample to diffuse.

In other embodiments of the invention, the first cover panel is detachably connected with the first bottom panel by adhering or welding or snapping with snaps provided at the edges and/or the corners of each panel. In a specific embodiment, the first cover panel comprises round apertures, and one microporous membrane is disposed between the first cover panel and the first bottom panel. When the first cover panel and the first bottom panel are fastened or engaged or adhered together, each round aperture on the first cover panel becomes a trough, i.e. testing trough, with a microporous membrane at the bottom, as shown in Figures 4E and F.

In an embodiment of the invention, the detection device further includes a sample containing component, comprising a second cover panel and a second bottom panel, wherein the surface of the second cover panel comprises cylindrical sample wells.

In other embodiments of the invention, the second cover panel is detachably or non-detachably connected with the second bottom panel by adhering or welding or snapping with snaps provided at the edges and/or the corners of each panel.

In an embodiment of the present invention, the side of the first cover panel is non-detachably connected with the side of the second cover panel to form an integrated panel. The side of the first bottom panel is non-detachably connected with the side of the second bottom panel to form another integrated panel.

In an embodiment of the present invention, a flow guiding groove is disposed between the first cover panel and the second cover panel. The flow guiding groove is a long groove perpendicular to the direction line that connects the centers of the first cover panel and the second cover panel. The shape of the cross section of the flow guiding groove is an inverted triangle or an arc. The flow guiding groove is disposed to prevent the liquid overflowing from the sample wells from entering into the testing troughs to interfere with detection result. If the liquid sample overflows or spills from the sample wells, it can flow into the flow guiding groove along the surface of the second cover panel and then be stored in the flow guiding groove or flows out along the flow guiding groove, instead of entering into the first cover panel. Further, when there are more than one sample wells, a flow guiding groove may be disposed in the middle of each two adjacent sample wells to prevent cross-contamination.

In another embodiment of the present invention, the side of the first cover panel is detachably connected with the side of the second cover panel, or the side of the first bottom panel is detachably connected with the side of the second bottom panel.

In another embodiment, the side of the first cover panel is detachably connected with the side of the second cover panel, and the side of the first bottom panel is detachably connected with the side of the second bottom panel.

In a more preferred embodiment, the connection means connecting by a movable component, for example by a soft material or by a hinge, whereby the sample containing component can be folded with the testing component.

In one embodiment of the present invention, the shape of the sample containing component or the testing component may be of any shape, preferably triangular, quadrangular, polygonal, oval or round, and more preferably, axially symmetric.

In a specific embodiment, the number of the sample wells is the same as the number of the testing troughs.

In one embodiment of the present invention, the sample well and the testing trough are arranged in a single row, or in a plurality of rows, or in a polygonal arrangement, or in a circular arrangement, or in an arbitrary arrangement, preferably in a straight line arrangement.

In another embodiment of the invention, the sample wells and testing troughs are arranged in the same manner.

In some embodiments of the invention, the sample wells and/or the testing troughs may be of any shape, preferably triangular, a quadrangular, a polygonal, oval or round shape.

In a specific embodiment, the sample wells and testing troughs are round-shaped.

In some embodiments of the invention, the sample well has a diameter of 0.3 to 1 cm, most preferably 0.5 cm, and a depth of 0.3 to 1 cm, most preferably 0.5 cm.

In some embodiments of the invention, the testing trough has a diameter of 0.5 to 2 cm, most preferably 1.8 cm, and a depth of 0.2 to 1 cm, most preferably 0.3 cm.

In a specific embodiment, the sample wells and testing troughs are in the same size.

In one embodiment of the invention, the sample applicator includes a hollow box-like member containing capillary tubes.

In some embodiments of the invention, the hollow box-like member is detachably or non-detachably connected with the sample containing component and/or the testing component. The hollow box member having an opening for facilitating access to the capillary tubes. Preferably the opening, the sample wells and the testing troughs are oriented in the same direction. Preferably, the opening is provided with a cover.

In some embodiments, the sample applicator includes a solid box-like member provided therein recessed holes for anchoring capillary tubes.

In some embodiments, the solid box-like member is detachably or non-detachably connected with the sample containing component and/or the testing component. Preferably, the solid box-like member is integral with the sample-containing component and/or the testing component. More preferably, a recessed hole is provided directly in the sample containing component and/or the testing component for anchoring capillary tubes. Preferably, there's only one recessed hole therein can anchor all the capillary tubes. Preferably, one recessed hole can anchor a plurality of capillary tubes. More preferably, the recessed holes has the same number as that of the capillary tubes, and each has a diameter and a depth of slightly larger than the outer diameter and the length of a capillary tube, thereby each recessed hole can anchor one capillary tube. The recess holes do not intersect with the sample wells or the testing troughs.

In one embodiment, the sample applicator comprises a body portion and capillary tubes disposed on the side. The body portion may be a beam or cuboid with channels therein penetrating through the body portion for anchoring capillary tubes. Thereby, when a capillary tube is anchored in channel, both ends of the capillary tube are open. In a specific embodiment of the present invention, the capillary tubes and the cuboid are integral as a whole. Correspondingly, the sample containing component and/or the testing component are provided with recessed holes into which the capillary tube of the sample applicator can be anchored.

In a specific embodiment, the number of capillary tubes equal to that of the sample wells or testing troughs. They may have the same spatial arrangements. In a specific embodiment, 2 or more, e.g. 3, capillary tubes are disposed on and extend from the same side of the cuboid member, thereby the sample applicator includes an array of parallel capillary tubes.

In a specific embodiment of the present invention, the sample applicator further comprises anchoring pin extending from the same side as the capillary tubes. Both the first cover panel and the second cover panel have anchoring holes. When the anchoring pins are inserted into the anchoring holes of the second cover panel, the capillary tubes are inserted into the sample wells to draw the liquid in the wells. When the anchoring pins are inserted into the anchoring holes of the first cover panel, the orifice of the capillary tubes are in full and close contact with the microporous membrane in the testing troughs, thereby the liquid in the capillary tubes is adsorbed by and diffuse into the microporous membranes.

In one embodiment of the invention, each anchoring pin may be in a shape of a bullet, cylinder-, rectangular column-, triangular prism, irregular column, arbitrary column shape, or inclined column. In a specific embodiment of the invention, each anchoring pin is in the shape of a bullet.

In one embodiment of the invention, the anchoring pins are hollow.

In one embodiment of the invention, the anchoring pins are positioned on each side of the sample applicator or in between the capillary tubes.

In one embodiment of the invention, the number of anchoring pins is at least 2, preferably 2, 3, 4 or more.

In a specific embodiment of the invention, the number of anchoring pins is 2 and respectively disposed on both sides of the sample applicator.

In a specific embodiment of the present invention, the sample containing component and/or the testing component have a plurality of recessed holes to accommodate the capillary tubes and anchoring pins on the sample applicator. The recessed holes are spatially arranged such that capillary tubes and anchoring pins of a sample applicator can be inserted into the holes and thus the sample applicator is detachably attached to the sample containing component or the testing component.

In a specific embodiment of the invention, the sample applicator is provided with projections or grooves for the purpose of increasing friction and facilitating handling, insertion or removal of the sample applicator.

In some embodiments of the invention, one end of each capillary tube is connected to a means for generating a negative pressure inside the capillary tube. In one embodiment of the present invention, the means for generating a negative pressure inside the capillary tube is a rubber ball. In use, pinch the rubber ball before or after the other end of the capillary tube is inserted into the liquid. Hold the capillary tube in the liquid and then release the rubber ball to generate a negative pressure in the capillary tube. After the negative pressure is generated, the liquid will enter into the capillary tube. n a preferred embodiment of the invention, the means for generating a negative pressure inside the capillary tube is a piston device such as a syringe, a pipette or the like. In use, the other end of the capillary tube is first inserted in the liquid, and the piston is pulled manually or by the elastic means, a negative pressure is generated inside the capillary tube, allowing the liquid sample to enter into the capillary tube more quickly. In an embodiment of the present invention, the above-described means for generating a negative pressure inside the capillary tube can also generate a positive pressure inside the capillary tube to push the liquid in the capillary slowly flow out and release into the microporous membrane, thereby the detection is completed.

In some embodiments of the invention, the sample containing component, the testing component and the sample applicator are made of a PVC material, a PUC material, a nylon material, a rubber material, an acrylonitrile-butadiene-styrene copolymer materials (ABS materials), a glass or a metallic material. Preferably, the metallic material is alloy material, more preferably, stainless steel.

The detection device of the present invention has at least two core components, a testing component having one or more microporous membrane, and a sample applicator having one or more capillary tubes. Therefore, those skilled in the art will understand the preparation method according to the disclosure of the specification. For the testing component, the microporous membranes can be fixed on a supporting surface by the three methods shown in Figure 4 or by other methods. For the sample applicator, if there is more than one capillary tube, for example 3, the capillary tubes can be connected by a connecting bridge, as shown in Figure 1C. Each of the capillary tubes includes an orifice for releasing liquid and another open outlet. The arrangement of the capillary tubes can be in any other manner.

### The Detection Kit

Another aspect of the present invention provides a kit for determining whether a biological sample contains misfolded proteins, including a microporous membrane and a capillary tube.

In some embodiments of the invention, the kit comprises any of the detection devices described above.

In a specific embodiment of the invention, the detection device of the kit comprises a plate member having therein at least a first row of wells and a second row of troughs, and a sample applicator embedded in and operably detachable from the plate member. The number of the wells or the number of the troughs is 1 or more (3, 10, 12 or greater). The wells are sample wells and the troughs are testing troughs. Preferably, the plate member comprises 3 or more sample wells, including at least one test sample well (containing a dye above or a dye solution above or a mixture of a dye above and a test sample), one negative control sample well (containing at least 30 µL of negative control sample) and one positive control sample (containing at least 30 µL of positive control sample). The sample wells can be sealed with a sealing sheet. The bottom of each testing trough is covered with an above microporous membrane such as filter paper. The applicator embedded in and operably detachable from the plate member comprises one or more capillary tubes. Two or more capillary tubes that have same lengths are fixed to a beam in parallel to each other, so that the spacing between any two adjacent capillary tubes on the beam is equal to the spacing between any two adjacent sample wells and the spacing between any two adjacent testing troughs, thereby the plurality of capillary tubes can be simultaneously inserted into the corresponding sample wells to draw liquid and then simultaneously inserted into the corresponding testing troughs.

In a specific embodiment of the invention, the kit comprises a dye capable of binding to a microporous membrane and misfolded proteins. The dye is selected from one or more of a heterocyclic dye, Congo Red, thioflavin, and Evans blue. Preferably the dye is Congo Red. In a preferred embodiment of the invention, the dye is preloaded into the sample well as a solid dry powder or solution.

In specific embodiments of the invention, the kit further comprises control samples, including a positive control sample and/or a negative control sample. In a preferred embodiment of the invention, the control sample is preloaded into the control sample wells.

In a specific embodiment of the invention, the sample wells are sealed with a sealing sheet. The sealing sheet may be tin foil, plastic film, or aluminum foil. The sealing can be performed by gluing or molding or any other method that can be used to seal.

In a specific embodiment of the invention, the negative control sample contains no misfolded proteins and the positive control sample contains misfolded proteins. In some embodiments of the invention, the positive control sample comprises any material having the same or similar structure as the misfolded proteins, and having a property capable of competing with the microporous membrane for binding a dye (e.g., Congo Red), such as any protein having a β-sheet structure, more specifically, such as denatured bovine serum albumin (BSA). In a specific embodiment of the invention, the negative control sample is PBS buffer and the positive control sample is PBS buffer containing misfolded BSA. In another specific embodiment of the invention, the negative control sample is a urine sample that does not contain misfolded protein and the positive control is a urine sample containing misfolded proteins.

In some embodiments, the kit may further comprise the above-described reference card, desiccants and an instruction for use. In a specific embodiment of the invention, the biological sample is body fluid from a patient diagnosed with a misfolded protein-related disease, e.g., whole blood, serum, plasma, urine, saliva, sweat, cerebrospinal fluid, hydrothorax, tears, vaginal secretions, semen, tissue lysate or combinations thereof. When a sample contains blood and has color, it can be centrifuged to remove red blood cells or other interfering factors before tested. In a specific embodiment of the invention, the misfolded protein disease is preeclampsia and the sample is a pregnant woman's urine sample.

In order to make the technical solutions and beneficial effects of the present invention more clear, the present invention will be further described in detail below with examples. It is understood that the specific examples described herein are merely illustrative of the invention and are not intended to limit the invention.

### Example 1

This example provides the shapes of the capillary tubes used in the present invention. As shown in Figure 1A, the shapes of the capillary tubes used in the present invention may be in a variety of shapes. The cross-section of the orifice that is contacted with a microporous membrane (i.e., the liquid outlet) may also be of various shapes including, but not limited to, a circle, an ellipse, a regular polygon, an irregular polygon, a polygon with rounded corners, Figure 1B). These capillary tubes can draw 5-20 µL of liquid. When a capillary tube contains liquid and with an orifice in full and close contact with a filter paper, the speed of the liquid released into the filter paper is no more than 4 µL/sec.

When a plurality of, e.g. 3, capillary tubes are used at the same time, a tandem of capillary tubes may be used. As shown in Figure 1C, the capillary tubes are connected by a connecting bridge 108. The orifices 102 of the capillary tubes 100 are used for drawing liquid and contacting the filter paper. The other ends 106 penetrate the connecting bridge and are open to the air.

### Example 2

This example provides a kit comprising a device as shown in Figures 2, 3, or 4, and further comprising one or two stainless steel capillary tubes having an inner diameter of 1.25 mm.

The device shown in Figure 2 comprises a testing component 201 having a filter paper 251. There are 3 ways to dispose the filter paper on the testing component:
First: the testing component 201 is a rectangular parallelepiped with a round filter paper 251 covered on the upper surface (this disposition is as shown in Figures 4A, 4B).
Second: the testing component 201 is a rectangular parallelepiped with a cylindrical trough 231 on the upper surface. A filter paper 251 is disposed at the bottom of the well (Figures 4C, 4D).
Third: The testing component 201 comprises a cover panel 211 and a bottom panel 212. The cover panel includes a round hole 271. One filter paper 251 is disposed between the first cover panel 211 and the first bottom panel 212. When the first cover panel 211 and the first bottom panel 212 are adhered together, the round hole 271 becomes a trough, i.e. testing trough 231, with a filter paper 251 at the bottom (as shown in Figures 4E and 4F).

The device shown in Figure 3 comprises a testing component 301 which is a rectangular parallelepiped having two testing troughs 311 and 312 on the surface. Each of the testing troughs 311 and 312 is a cylindrical recessed hole having a depth of about 2/3 of the thickness of the rectangular parallelepiped. The bottom of each testing trough flatly covered with a round filter paper having a diameter slightly smaller than that of the cross section of the testing trough (the arrangement is shown in Figures 4C and 4D, the filter paper is not shown in Figure 3).

The specific detection steps for determining whether a pregnant woman's urine sample contains misfolded proteins comprising: mixing the urine sample from the pregnant woman with Congo Red to form a mixture, placing a capillary tube vertically in the mixture for about 10 seconds (drawing about 10 µL mixture), placing the capillary tube to the testing trough to be in full and close contact with the filter paper, keeping the capillary tube stay vertically for about 15 seconds, thereby the mixture in the capillary slowly released into the filter paper, and then removing the capillary tube and observing the diffusion result.

The device as shown in Figure 2 can be used to detect only one test sample. While the device as shown in Figure 3 may be used to detect 2 test samples, or to detect 1 test sample and 1 control sample. The control sample can be a negative control sample or a positive control sample. The negative control sample is PBS buffer and the positive control sample is PBS buffer containing misfolded BSAs formed by specially denatured treatment.

### Example 3

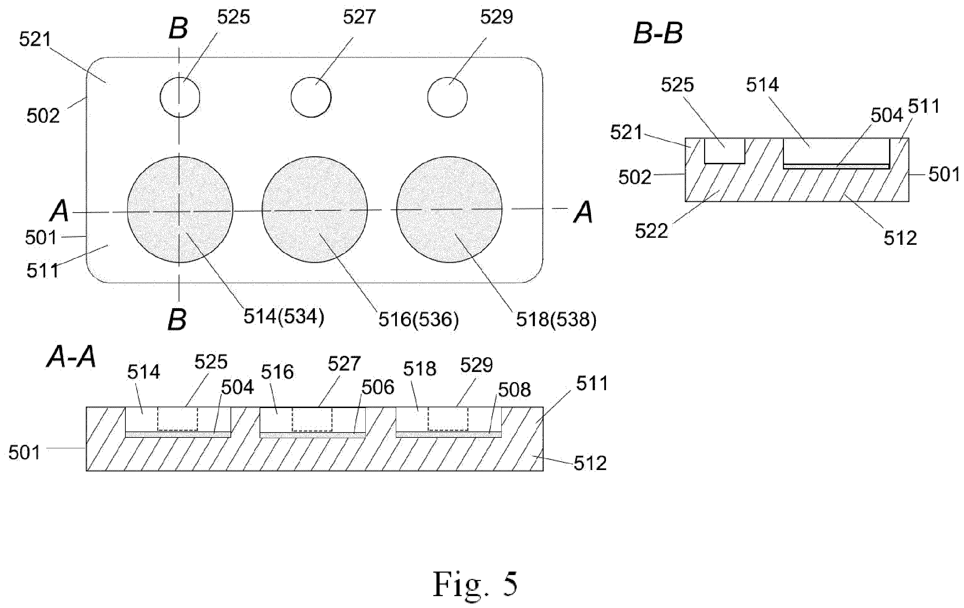

This example provides a kit and a method to use thereof. The kit comprises a device as shown in Figures 5, 6, 7, or 8

The testing device shown in Figure 5 includes a testing component 501 and a sample containing component 502. The testing component 501 comprises a first cover panel 511 and a first bottom panel 512. The surface of the first cover panel 511 is provided with three cylindrical troughs, i.e. testing troughs 514, 516 and 518, having at the bottom filter papers 534, 536, 538 disposed by the method as shown in Figs.4C and 4D, respectively. The lower surface of the first cover panel 511 is non-detachably connected to the upper surface of the first bottom panel 512 to form an integrated panel. The sample containing component 502 comprises a second cover panel 521 and a second bottom panel 522. The upper surface of the second cover panel is provided with three sample wells 525, 527, 529. The lower surface of the second cover panel 521 is non-detachably connected to the upper surface of the second bottom panel 522 to form an integrated panel. One side surface of the first cover panel 511 is directly connected to one side surface of the second cover panel 521 to form an integrated panel. One side surface of the second cover panel 512 is directly connected to one side surface of the second bottom panel 522 to form an integrated panel. Thereby, the testing component 501 and the sample containing component 502 are integral as a whole and cannot be physically distinguished.

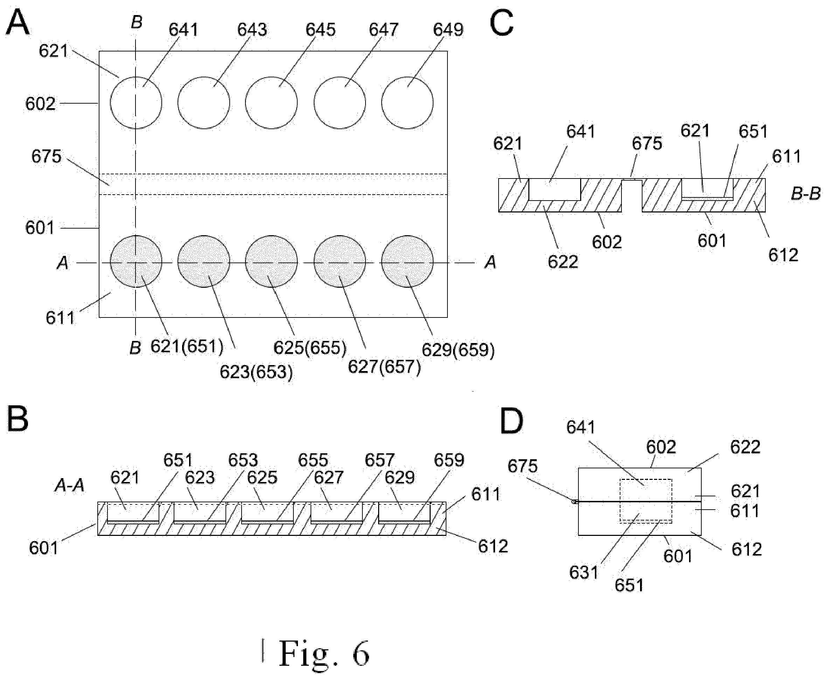

The detection device shown in Figure 6 comprises a testing component 601 and a sample containing component 602. The testing component 601 comprises a first cover panel 611 and a first bottom panel 612. The upper surface of the first cover 611 is provided with five testing troughs 631, 633, 635, 637 and 639 having at the bottom filter papers 651, 653, 655, 657 and 659, disposed by the method as shown in Figs.4C and 4D, respectively. The sample containing component 602 comprises a second cover panel 621 and a second bottom panel 622. The upper surface of the second cover panel is provided with five sample wells 641, 643, 645, 647, 649. One side surface of the first cover panel 611 is connected to one side surface of the second cover panel 621 by a soft material 675, and any side surface of the first bottom panel 612 is not connected to any side surface of the second bottom panel 622. Thereby, the testing component 601 and the sample containing component 602 can be folded together during transportation and storage to save space.

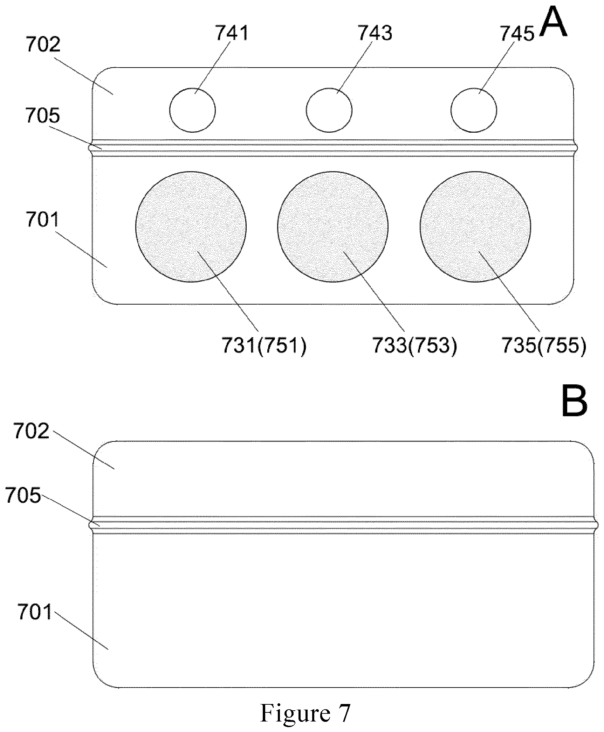

The detection device shown in Figure 7 comprises a testing component 701 and a sample containing component 702. The upper surface of the testing component 701 is provided with three testing troughs 731, 733 and 735 having at the bottom filter papers 751, 752 and 753, disposed by the method as shown in Figs. 4C and 4D, respectively. The upper surface of the sample containing component 702 is provided with three sample wells 741, 743, and 745. The testing component 701 and the sample containing component 702 are connected by a living hinge 705.

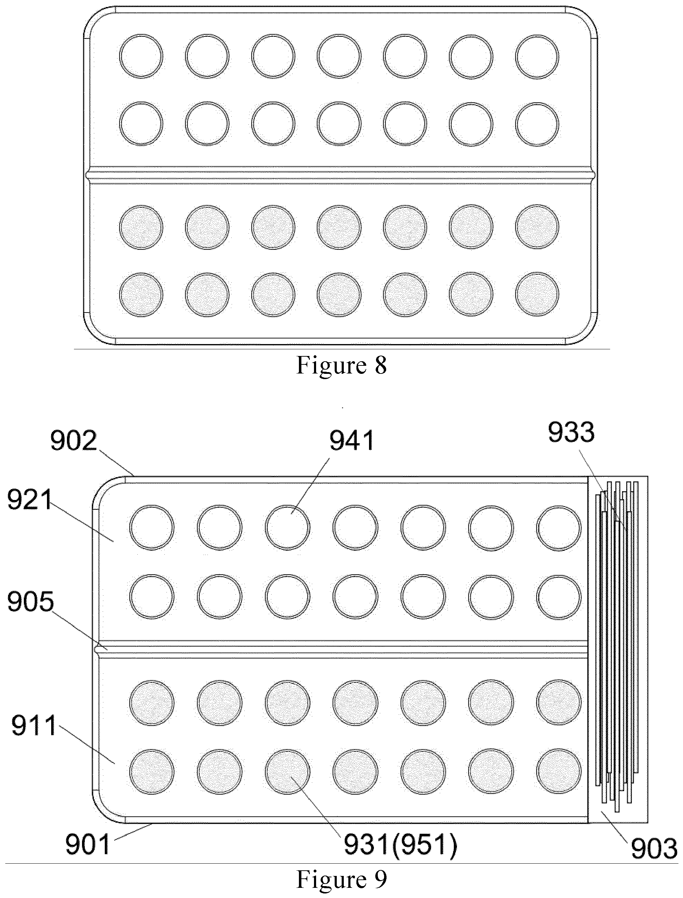

The detection device shown in Figure 8 is substantially identical to that shown in Figure 7 except that the numbers (14 and 14) of the testing troughs and the sample wells are greater than the numbers of that of the device shown in Figure 7, which can be used to detect more samples.

Each kit also includes the same number of stainless steel capillary tubes as that of sample wells, and they have an inner diameter of 1.25 mm.

The specific detection steps for determining whether a pregnant woman's urine sample contains misfolded proteins includes: mixing the urine sample with Congo Red (5 mg/mL) at a ratio of 20:1 to form a mixture. inserting a capillary tube vertically in the mixture for about 10 seconds (drawing about 10 µL mixture). placing the capillary tube to the testing trough to be in full and close contact with the filter paper. keeping the capillary tube stay vertically for about 15 seconds, and letting the mixture in the capillary tube slowly release into the filter paper. then removing the capillary tube and observing the diffusion result.

The device as shown in Figure 5 or Figure 7 may be used to detect only 3 test samples, or to detect, of course, one test sample, 1 negative control sample, and 1 positive control sample by the same detection method. The device as shown in Fig 6 can be used to detect up to 5 test samples, or to detect 3 test samples and 2 control samples, i.e., 1 negative control sample and 1 positive control sample. The device as shown in Figure 8 can be used to detect up to 14 test samples, or to detect 12 test samples, 1 negative control sample, and 1 positive control sample.

### Example 4

This embodiment provides a kit and a method to use thereof. The kit comprises a device as shown in Figure 9, Figure 10 or Figure 11.

The detection device shown in Figure 9 comprises a testing component 901, a sample containing component 902, and a sample applicator 903. The testing component 901 comprises a first cover panel 911 and a first bottom panel 912 (not shown). The upper surface of the first cover panel 911 is provided with 14 testing troughs 931. The sample containing component 902 comprises a second cover panel 921 and a second bottom panel 922 (not shown). the upper surface of the second cover panel 921 is provided with 14 sample wells 941. The first cover panel 911 and the first bottom panel 912 are integrated as a whole. The testing troughs 931 are provided with filter papers 951 at the bottom by the method as shown in Figure 4C and 4D. The second cover panel 921 and the second bottom panel 922 are integrated as a whole. The testing component 901 and the sample containing component 902 are connected by a snap 905, and can be detached separately and stacked together during transportation and storage and connected together when in use. The sample applicator 903 is a hollow box-like member containing glass capillary tubes 933 each having an inner diameter of 1.2 mm. The hollow box-like member is connected to the sample containing component 902 and the testing component 901 by snapping.

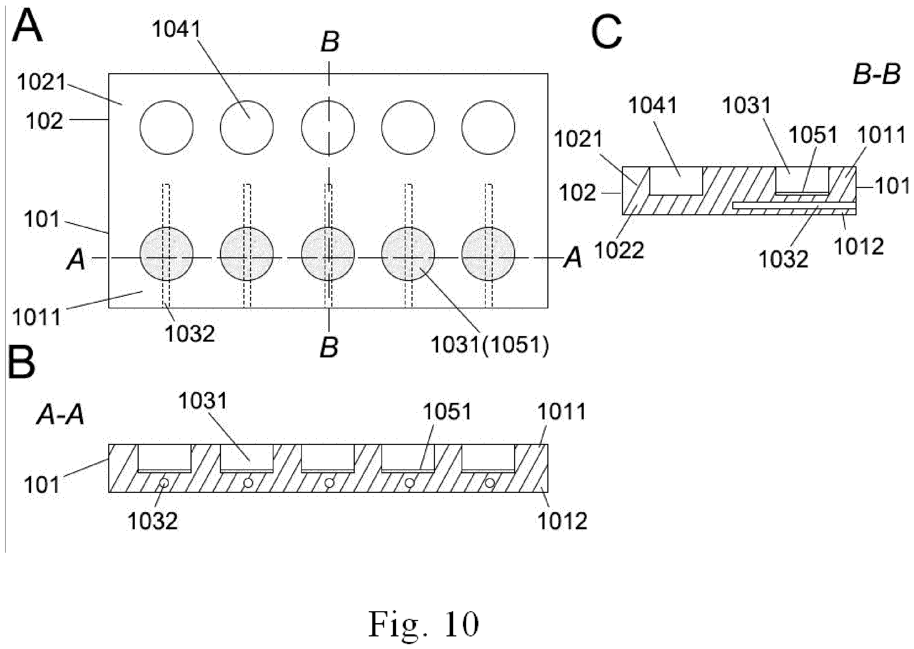

The detection device shown in Figure 10 comprises a testing component 1001, a sample containing component 1002, and a sample applicator 1003. The testing component 1001 comprises a first cover panel 1011 and a first bottom panel 1012. The upper surface of the first cover 1011 is provided with 5 testing troughs 1031. The sample containing component comprises a second cover panel 1021 and a second bottom panel 1022. The upper surface of the second cover panel 1021 is provided with 5 sample wells 1041. The first cover panel 1011 and the first bottom panel 1012 are integrated as a whole. The testing troughs 1031 are provided with filter papers 1051 at the bottom by the method as shown in Figs.4C and 4D. The second cover panel 1021 and the second bottom panel 1022 are integrated as a whole. Similarly, the first cover panel 1011 and the second cover panel 1021 are integrated as a whole, and the first bottom panel 1012 and the second bottom panel 1022 are integrated as a whole. That is to say, the testing component 1001 and the sample containing component 1002 are integrated as a whole. The sample applicator 1003 is a solid box-like member on which as many recessed holes 1032 as the sample wells 1041 are provided, each of the recessed holes 1032 has a diameter and depth slightly larger than those of the capillary tube (not shown). Each recessed hole can anchor one capillary tube. The upper surface of the solid box-like member is non-detachably connected to the lower surface of the first bottom panel 1012 and the lower surface of the second bottom panel 1022 thereby forms an integrated panel.

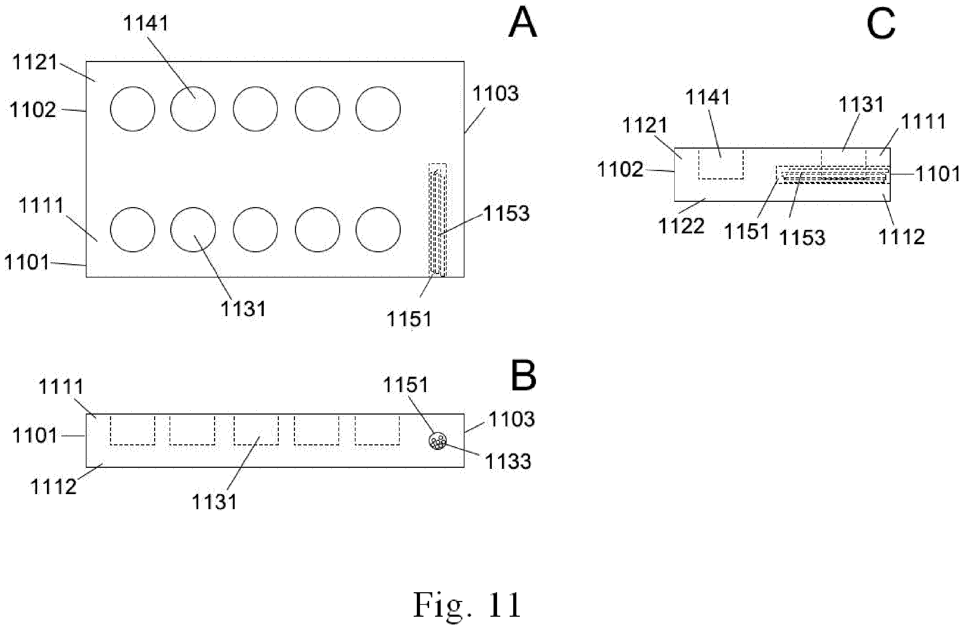

The testing device shown in Figure 11 comprises a testing component 1101, a sample containing component 1102, and a sample applicator 1103. The testing component 1101 comprises a first cover panel 1111 and a first bottom panel 1112. The upper surface of the first cover panel 1111 is provided with 5 testing troughs 1131. The sample containing component 1102 comprises a second cover panel 1121 and a second bottom panel 1122. The upper surface of the second cover panel 1121 is provided with five sample wells 1141. Like the detection device shown in Figure 10, the detection device is an integrated piece. The upper surface of the first cover panel 1111 is provided with 5 testing troughs 1131 having at the bottom filter papers 1151, disposed by the method as shown in Figs. 4C and 4D, respectively. The difference is that the sample applicator 1103 is connected to the side surface of the testing component 1101 and the sample containing component 1102. The sample applicator 1103 is provided with a recessed hole 1151 having a depth slightly greater than the length (about 3 cm) of the capillary tubes 1153. The recessed hole 1151 has a depth of lightly larger than the length of the capillary tubes 1153, and can anchor a number of stainless capillary tubes no less than the sample wells 1141. Each capillary tube has an inner diameter of 1.25 mm.

The steps of the detection method are the same as those in Examples 2 and 3.

### Example 5

This example provides a kit and a method to use thereof. The kit comprises a device as shown in Figures12, 13, or 14.

The detection device shown in Figure 12 comprises a testing component 1201, a sample containing component 1202 and a sample applicator 1203, all of which are made of a PVC material. The testing component 1201 comprises a first cover panel 1211 and a first bottom panel 1212 (not shown). The sample containing component 1202 comprises a second cover panel 1221 and a second bottom panel 1222 (not shown). The upper surface of the second cover panel 1221 is provided with 3 sample wells 1241. One side of the first cover 1211 is non-detachably connected to one side of the second cover 1221 to form an integrated panel. Between the first cover 1211 panel and the second cover pane 1221 is provided a flow guiding groove 1206, which is a long groove perpendicular to the direction line connecting the centers of the first cover panel 1211 and the second cover panel 1221. The shape of the cross section of the flow guiding groove is an inverted triangle. One side surface of the first bottom panel 1212 is non-detachably connected to one side surface of the second bottom panel 1222 to form an integrated panel. The upper surface of the first cover panel 1211 is provided with 3 circular holes. When the first cover panel 1211 is connected to the first bottom panel 1212, a layer of filter paper 1251 is disposed in the middle (as shown in Figures 4E, 4F). Thus, after the first cover panel 1211 and the first bottom panel 1212 snapping together by the snaps at the corners of the panels, the circular holes form the testing troughs 1231 having at the bottoms the filter paper 1251.

The sample applicator 1203 is a sample applying member, and capillary tubes 1233 are disposed on the side. The capillary tubes 1233 has a same number as that of the sample wells 1241 or the testing troughs 1231, and has a same arrangement as that of the sample wells 1241 or the testing troughs 1231. The surface of the applicator on which the capillary tubes 1233 disposed further is provided with positioning pins 1235. The upper surface of the first cover panel 1211 and the upper surface of the second cover panel 1221 are provided with positioning holes 1204 and 1208, respectively. When the positioning pins 1235 are inserted into the positioning hole 1204 or 1208, the capillary tubes 1233 are inserted into the testing troughs 1231 or sample wells 1241. The side surface of the sample containing member 1202, with respect to the side surface that connects to the testing component 1201, is further provided with recessed holes(not shown) arranged in the same manner as the capillary tubes 1233 and the positioning pins 1235 on the sample applying member. In such way, the sample applying member can be coupled to the sample containing component 1202 or the testing component 1201. The sample applying member is further provided with projections 1237 for facilitating insertion or removal of the sample applying member.

The sample wells 1231 are preloaded with Congo Red. One of the sample wells is also preloaded with a positive control sample (PBS buffer containing BSA), and another sample well is preloaded with a negative control sample (PBS buffer without BSA). All sample wells are sealed with tin foil.

The detection device shown in Figure 13 is substantially identical to that shown in Figure 12, except that the numbers (5 and 5) of the testing troughs and the sample wells are greater than the numbers of those of the device shown in Figure 12, and thus can be used to detect more samples.

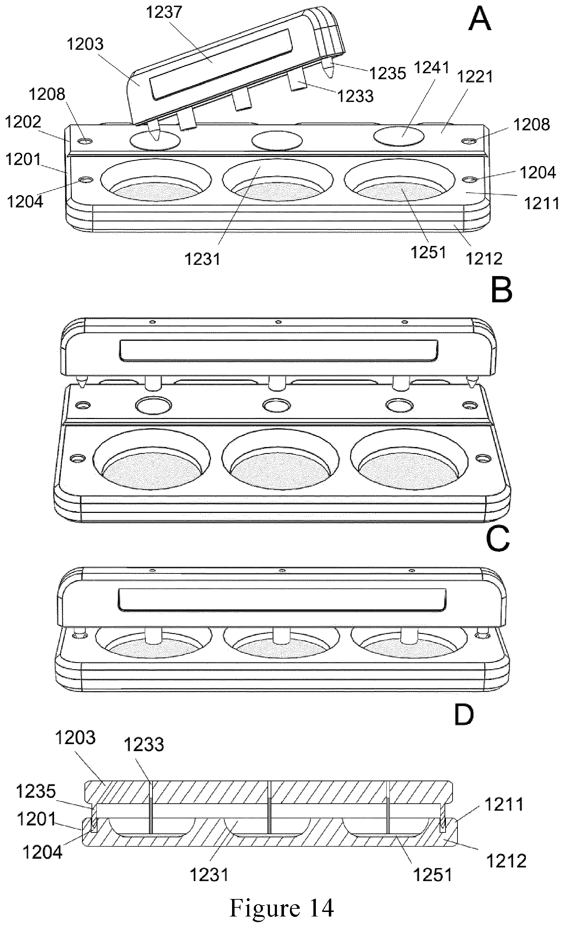

The steps of the method to use the kit as follows:
1. Detaching the sample applicator and placing the detection device body (comprising the testing component and sample containing component) on a table top horizontally;
2. Puncturing the tin foil on the sample wells using a positioning/anchoring pin (Figure 14A);
3. Adding the urine sample of a pregnant woman to the test sample well;
4. Inserting the positioning pins of the sample applicator into the positioning holes of the second cover panel (Figure 14B), whereby the capillary tubes on the sample applicator is inserted into the liquid in the sample wells, then the liquid enter automatically into the capillary tubes;
5. Inserting the positioning pins of the sample applicator into the positioning holes of the first cover panel (Figure 14C), whereby the capillary tubes on the sample applicator directly contact the microporous membrane deposed in the testing trough (as shown in the cross-sectional view of Figure 14D), keeping the sample applicator stay for 5-10 seconds;
6. Removing the sample applicator and then observing the detection result.
7. If the result is as shown in Figure 15A, i.e., a red blot is formed as the dye accumulates around the orifice of the capillary tube and does not diffuse (Figure 15A, a), or a red blot with "pseudopodia" is formed as the dye accumulates around the applying site and does not diffuse (Figure 15A, b), or a red blot with a slight diffusion is formed (Figure 15A, c), then the urine sample of the pregnant woman is determined to be negative, i.e., does not contain misfolded proteins or contains misfolded proteins of an amount smaller than a reference value.

If the result is as shown in Figure 15B, i.e., a small red blot with a relatively obvious diffusion is formed around the applying site (Figure 15B, d), or a red blot with irregular light diffusion is formed around the applying site (Figure 15B, e), or a uniform, larger round diffusion blot is formed around the applying site (Figure 15B, f), then the urine sample of the pregnant woman is determined to be positive, i.e., contains misfolded proteins or contains misfolded proteins of an amount larger than a reference value.

The detection device provided by this example integrates the device and the reagents, greatly compressing the packaging space, and is convenient for transportation, storage, and is convenient for laboratory operation.

### Example 6

This example provides a simple kit and a method to use thereof. The kit comprises a filter paper and a stainless capillary tube having an inner diameter of 1.25 mm. The steps of the method are as follows:
1. Placing the filter paper horizontally on the table top;
2. mixing the urine sample from a pregnant woman with Congo Red to form a mixture;
3. drawing the mixture with the capillary tube, placing the orifice of the capillary tube in full and close contact with the filter paper, keeping the capillary tube stay for more than 10 seconds, thereby the mixture in the capillary tube is slowly released into the filter paper,
4. waiting for about 10 seconds and then observing the detection result.

Figure 16 is a view showing the detection process of the detection device of the present invention. After a capillary tube is contacted with the filter paper, the liquid is slowly released into the filter paper and diffuses outward from the applying site to form colored blots of different sizes.

### Example 7

In order to compare the effect of application methods, a capillary tube, a pipetteman and a dropper are compared. The inventors designed the following experiments:

### 1. Capillary tube, pipette and dropper

The capillary tube used was a glass capillary tube having a circular orifice with an inner diameter of 1.25mm, a length of 10 cm, and a 10 µL marker thereon.

The pipette used was a 10 µL pipetteman.

The dropper used was a 100 µL dropper with 10 µL of liquid drawn per detection.

### 2. The negative sample and the positive sample

The negative sample is the urine sample of a pregnant woman without diagnosis of preeclampsia according to the clinical result. The positive sample is the urine sample of a pregnant woman diagnosed with preeclampsia according to the clinical result.

### 3. Detection

Congo Red was added into the negative sample and the positive sample at the ratio of 50:1. The capillary tube was inserted into the negative sample or the positive sample. After the liquid level in the capillary tube rises to the 10 µL mark, the capillary tube was placed onto the filter paper. After the liquid in the capillary tube was released into the filter paper completely, the capillary tube was removed. The detection result was observed after 15 seconds.

Similarly, 10 µL of the negative sample or positive sample was also drawn using a pipetteman (with a tip) or a dropper. Then the pipette tip or the dropper tip was placed in full and close contact with the filter paper to release the liquid into the filter paper. The detection result was observed after diffusing for a while.

In order to compare the difference between the sample applying methods: contacting to slowly release the liquid or dropping the liquid, 10 µL of the negative sample or positive sample was drawn by a capillary tube, a pipetteman and a dropper, respectively. The capillary tube, the pipette and the dropper was held 0.5 to 2 cm above a filter paper, and the liquid was dropped onto the filter paper. Then the detection result was observed after the liquid diffuses.

The radiuses of the diffusion blots of the negative sample and the positive samples were measured, respectively. The radius of a diffusion blot is the length from the center of the blot to the point where the blot diffused to the farthest position. And the radius ratio of the radius of the diffusion blot of the positive sample to the radius of the diffusion blot of the negative sample was calculated. Each experiment was repeated 5 times.

### 4. Results

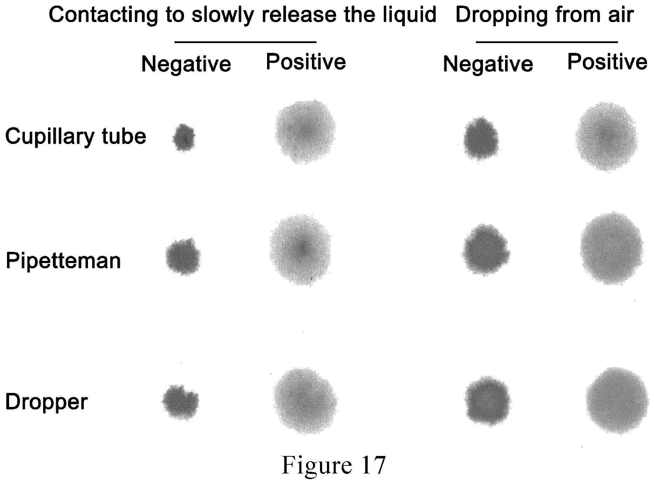

The diffusion blots formed by applying with capillary tube, pipetteman and dropper are shown in Figure 17 and Table 1.

### For positive samples:

Regardless of the applicator and the sample applying method, the positive samples formed a uniform, large diffusion blots with almost no difference.

### For negative samples:

If the liquid was dropped onto the filter paper using the three applicators (capillary tube, pipette gun and dropper), the negative samples form relatively large blots, having relatively small differences from the blots formed by positive samples using the same sample applying method. The radius ratios are about 1.2, which makes it easy to make a false determination.

If the liquid was released by contacting to the filter paper, a dark blot with no or little diffusion was observed upon using a capillary tube. The radius ratios are above 2.5. In contrast, the blot of the negative sample using pipetteman or dropper is relatively large and with irregular diffusion. And the radius is about 1.6.

**Table 1 Effect of sample application methods on blot diffusion (n=5, p<0.01)**

| | Contacting to slowly release the liquid | | | Dropping from air | | |
|---|---|---|---|---|---|---|
| | Radius of a diffusion blot (mm) | | Radius ratio | Radius of a diffusion blot (mm) | | Radius ratio |
| | Negative | Positive | | Negative | Positive | |
| Capillary tube | 2.10±0.04 | 5.56±0.30 | 2.65±0.15 | 4.02±0.07 | 5.44±0.21 | 1.36±0.07 |
| Pipetteman | 3.34±0.14 | 5.63±0.30 | 1.69±0.14 | 4.37±0.21 | 5.45±0.28 | 1.25±0.04 |
| dropper | 3.41±0.21 | 5.50±0.25 | 1.62±0.04 | 4.54±0.19 | 5.34±0.29 | 1.20±0.10 |

In summary, the detection method that contacts filter paper to slowly release the liquid using capillary tubes is surprisingly the best. The detection results are obvious to distinct. It is not easy to form false determination, and the determinations are intuitive and reliable.

### Example 8

In order to compare the effects of different inner diameters of the capillary tubes on the detection results, the inventors designed the following experiments:

### 1. Capillary tubes

The capillary tubes used were stainless steel capillary tubes with inner diameters of 0.4, 0.5, 0.6, 0.7, 0.9, 1.12, 1.25, 1.45, 1.69, 1.99, 2.4, 2.64 and 2.8 mm and a length of 3 cm, and each with a 10 µL mark thereon.

### 2. The negative sample and the positive sample

The negative sample is the urine sample of a pregnant woman without preeclampsia according to the clinical result. The positive sample is the urine sample of a pregnant woman diagnosed with preeclampsia according to the clinical result.

### 3. Detection

Congo Red was added in the negative samples and the positive sample, respectively. The capillary tubes with different inner diameters were inserted into the negative sample or positive sample, and were held for more than 30 seconds. When the liquid level was no longer rising, the orifices of the capillary tubes were placed in full and close contact with a filter paper until the liquid was released into the filter paper completely (about 3-10 seconds). Then the capillary tubes were removed from the filter paper. After the blot being diffused for a while, the detection results were observed.

The radiuses of the diffusion blots of the negative sample and the positive samples were measured, respectively. The radius of a diffusion blot is the length from the center of the blot to the point where the blot diffused to the farthest position. And the radius ratios of the radiuses of the diffusion blots of the positive sample to the radiuses of the diffusion blots of the negative sample were calculated. Each experiment was repeated 5 times.

### 4. Results

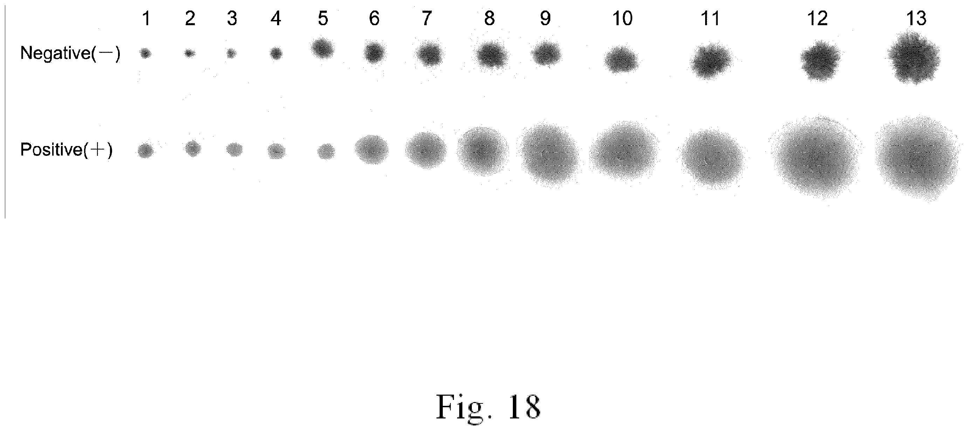

The diffusions of blots using capillary tubes with different inner diameter are shown in Figure 18 and Table 2. It can be seen that if the inner diameter of the capillary tube is too small, e.g. less than 0.7 mm, the speed of the liquid release from the capillary tube into the filter paper is too slow, thereby the blot diffusion radius of the positive sample is too small and does not differ much from that of the negative sample. As the inner diameter of the capillary tube increases, the blot diffusion radius of the negative sample does not increase significantly, but the blot diffusion radius of the positive sample increases rapidly. When the inner diameter of the capillary tube increases to a certain extent, e.g. greater than 3 mm, the blot diffusion radius of the negative sample also begins to increase since the contact area of liquid with the filter paper is too large. However, the blot diffusion radius of the positive sample does not change significantly, resulting in decreasing instead of increasing of the radius ratio. As the inner diameter of the capillary tube further increases, the liquid can no longer enter into the capillary tube, resulting in failure to detect. In this example, when the inner diameter of the capillary tube is 0.7-2.8mm, the detection result is good, and when the diameter is 1.12-2.64mm, the detection is better.

**Table 2 Effect of different capillary tube inner diameters on blot diffusion (n=5, p<0.01)**

| No. | Inner diameter (mm) | Time to be release completely (sec) | Radius of a diffusion blot (mm) | | Radius ratio |
|---|---|---|---|---|---|
| | | | Negative | Positive | |
| 1 | 0.40 | 9.06±0.65 | 1.00±0.07 | 1.66±0.1 | 1.67±0.17 |
| 2 | 0.50 | 8.31±0.40 | 1.03±0.06 | 1.61±0.1 | 1.56±0.09 |
| 3 | 0.60 | 8.67±0.39 | 1.02±0.06 | 1.53±0.1 | 1.51±0.04 |
| 4 | 0.70 | 7.44±0.37 | 1.44±0.08 | 2.95±0.19 | 2.05±0.20 |
| 5 | 0.90 | 5.21±0.42 | 1.91±0.07 | 4.17±0.24 | 2.19±0.11 |
| 6 | 1.12 | 4.86±0.34 | 1.97±0.14 | 4.86±0.28 | 2.48±0.26 |
| 7 | 1.25 | 4.64±0.22 | 2.20±0.08 | 6.49±0.08 | 2.95±0.10 |
| 8 | 1.45 | 3.92±0.28 | 2.58±0.16 | 7.1±0.36 | 2.76±0.24 |
| 9 | 1.69 | 3.70±0.24 | 2.47±0.12 | 7.37±0.44 | 2.98±0.21 |
| 10 | 1.99 | 3.25±0.21 | 2.66±0.18 | 7.65±0.6 | 2.88±0.3 |
| 11 | 2.4 | 2.72±0.14 | 2.65±0.12 | 7.45±0.47 | 2.81±0.18 |
| 12 | 2.64 | 2.47±0.17 | 2.93±0.20 | 7.41±0.60 | 2.54±0.25 |
| 13 | 2.80 | 2.20±0.13 | 3.34±0.19 | 7.49±0.25 | 2.25±0.09 |

### Example 9

In order to compare the effect of different sample applying amounts on the detection results, the inventors designed the following experiments:

### 1. Capillary tubes

The capillary tube used was a glass capillary tube with an inner diameter of 1.25 mm and a length of 5 cm. The maximum volume of liquid the capillary tube can draw is 20 µL. The capillary tube was marked with a scale of 1-20 µL with an accuracy of 1 µL.

### 2. The negative sample and the positive sample

The negative sample is the urine sample of a pregnant woman without preeclampsia according to the clinical parameters. The positive sample is the urine sample of a pregnant woman diagnosed with preeclampsia according to clinical parameters.

### 3. Detection

Congo Red was added into the negative sample and positive sample, respectively. The capillary tubes were inserted into the negative sample or the positive sample, respectively, to draw 2, 3, 4, 5, 6, 7, 8, 9, 10, 11, 12, 13, 14, 15, 16 or 17 µL of liquid. Then the capillary tubes were placed to fully and closely contact with filter papers until the liquid was released into the filter paper completely. Then the capillary tubes were removed and the detection results were observed.

The radiuses of the diffusion blots of the negative sample and the positive samples were measured, respectively. The radius of a diffusion blot is the length from the center of the blot to the point where the blot diffused to the farthest position. And the radius ratios of the radiuses of the diffusion blots of the positive sample to the radiuses of the diffusion blots of the negative sample were calculated. Each experiment was repeated 5 times.

### 4. Results

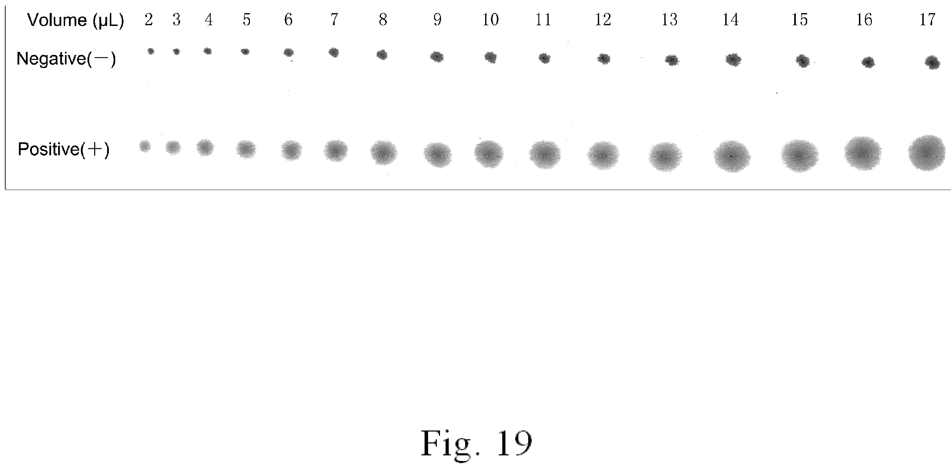

The diffusions of blots formed by different sample volumes are shown in Figure 19 and Table 3. It can be seen that as the sample volume increases, the blot diffusion radius of the negative sample does not increase significantly. However, the blot diffusion radius of the positive sample increases rapidly. The distinction between the blot diffusion of the negative sample and the positive sample is becoming more and more obvious since the radius ratio is gradually increasing. However, when the volume reaches 16 µL, the blot diffusion radius of the positive sample increased slowly or even no longer increased, which is affected by the diffusion mechanics, so that the blot is concentrated in a certain region. On the other hand, as the sample volume increases, the time for the capillary tube to draw liquid becomes longer and longer. Since the negative sample does not diffuse or diffuses to a small extent, it is difficult or takes longer for the liquid in the capillary tube to be completely released into the filter paper, resulting in a very long detection time. Considering the combination, the sample volume is at least 4 µL. The detection result is best when the sample volume is 5-15 µL.

**Table 3 The effect of sample volume applied on blot diffusion (n=5, p<0.01)**

| Sample volume (µL) | Radius of diffusion blot (mm) | | Radius ratio |
|---|---|---|---|
| | Negative | Positive | |
| 2 | 1.49±0.13 | 2.15±0.11 | 1.45±0.15 |
| 3 | 1.49±0.09 | 2.50±0.18 | 1.68±0.16 |
| 4 | 1.54±0.05 | 3.02±0.22 | 1.96±0.14 |
| 5 | 1.49±0.07 | 3.53±0.20 | 2.38±0.23 |
| 6 | 1.49±0.09 | 4.07±0.23 | 2.74±0.22 |
| 7 | 2.00±0.12 | 4.48±0.24 | 2.25±0.20 |
| 8 | 1.96±0.12 | 5.01±0.33 | 2.57±0.29 |
| 9 | 2.22±0.17 | 5.36±0.32 | 2.42±0.21 |
| 10 | 2.53±0.14 | 5.91±0.20 | 2.34±0.12 |
| 11 | 2.56±0.16 | 6.00±0.41 | 2.35±0.25 |
| 12 | 2.53±0.16 | 6.74±0.20 | 2.68±0.18 |
| 13 | 2.60±0.19 | 6.22±0.18 | 2.41±0.22 |
| 14 | 2.72±0.12 | 6.71±0.37 | 2.47±0.18 |
| 15 | 2.82±0.17 | 6.90±0.27 | 2.45±0.21 |
| 16 | 2.95±0.12 | 7.22±0.41 | 2.45±0.12 |
| 17 | 2.99±0.17 | 7.25±0.5 | 2.43±0.19 |

The above examples are used herein to demonstrate preferred embodiments of the invention. Those skilled in the art will appreciate that the techniques disclosed in the above examples are representative of the techniques discovered by the inventors that can be used to practice the invention and are therefore considered as a preferred embodiment of the invention. However, it will be apparent to those skilled in the art will appreciate that specific embodiments disclosed in the above examples can be modified a lot, still can obtain the same or the similar results without departing from the spirit or scope of the invention.

Unless defined otherwise, all technical and scientific terms used herein have the same meaning as commonly understood by those skilled in the art to which the invention pertains, and the materials cited herein and the materials they reference are incorporated by reference.

Many equivalents to the specific embodiments of the inventions described herein will be apparent to those skilled in the art. These equivalents will be included in the claims.

All documents mentioned in the present application are hereby incorporated by reference in their entireties as if the disclosures in In addition, it is to be understood that various modifications and changes may be made by those skilled in the art in the form of the appended claims.

## Claims

1. A device for determining whether a biological sample contains misfolded proteins, comprising:
a capillary tube having a liquid outlet with a cross-sectional area of no more than 9 mm² and capable of drawing and containing at least 5 µL of a liquid solution;
a microporous membrane capable of competing with misfolded proteins for binding to a dye,
wherein said liquid outlet of the capillary tube is in close contact with said microporous membrane.

2. The device of Claim 1, wherein said capillary tube contains at least 5µL mixture formed by mixing said dye and said biological sample, wherein said dye is selected from the group consisting of a heterocyclic dye, Congo Red, thioflavin, and Evans Blue.

3. The device of Claim 2, wherein the surface of the microporous membrane around said liquid outlet is stained by the mixture released from the capillary tube.

4. A device for determining whether a biological sample contains misfolded proteins, comprising:
a plate member having therein at least a first row of sample wells and a second row of troughs, and an applicator embedded in and operably detachable from the plate member, wherein
the first row of sample wells comprises a test sample well, a negative control sample well, and a positive control sample well, each of said sample wells being capable of holding 30-500 µL or more of liquid;
said second row of troughs comprises at least three testing troughs each having at the bottom a microporous membrane capable of competing with misfolded proteins for binding to a dye;
said applicator comprises at least three capillary tubes fixed to a beam in parallel to each other, so that the spacing between any two adjacent said capillary tubes on said beam is equal to the spacing between any two adjacent sample wells and the spacing between any two adjacent testing troughs thereby said three capillary tubes can be simultaneously inserted into the three sample wells or the three testing troughs, respectively, and
each of said capillary tubes has a liquid outlet having a cross-section area of no more than 9 mm² and capable of drawing and containing at least 5 µL of liquid.

5. The device of Claim 4, wherein said sample well is filled with a dye or dye solution capable of specifically binding to a misfolded protein, said negative control sample well is filled with a negative control sample, said positive control sample well is filled with a positive control sample, and wherein all the sample wells are sealed with a sealing sheet.

6. A kit for determining whether a biological sample contains misfolded proteins, comprising:
a capillary tube having a liquid outlet with a cross-section area of no more than 9 mm² and capable of drawing and containing at least 5 µL of liquid; and
a microporous membrane capable of competing with misfolded proteins for binding to a dye.

7. The kit of Claim 6, further comprising a reference card printed with examples of diffusion results of at least one negative sample and at least one positive sample.

8. A kit for determining whether a biological sample contains misfolded proteins, comprising the device of Claim 4 or 5.

9. A system for determining whether a biological sample contains misfolded proteins, comprising:
a device of Claim 4 or 5;
a signal collection module comprising an optical signal collector;
a signal memory module for storing processed or unprocessed signals collected by the signal collection module.

10. A method of determining whether a biological sample contains misfolded proteins, comprising the steps of:
mixing the biological sample with a dye capable of binding to a microporous membrane and a misfolded protein to form a mixture;
drawing at least 4 µL of the mixture with a capillary tube having a liquid outlet with a cross-sectional area of no more than 9 mm² and capable of drawing and containing at least 5 µL of liquid;
placing said liquid outlet of the capillary tube in full and close contact with the surface of the microporous membrane to release the mixture in the capillary tube into the microporous membrane;
determining whether the biological sample contains misfolded proteins by observing the diffusion of the mixture in the microporous membrane based on the color of the dye.
